# FASCICULE DE BREVET EUROPEEN

(11) **EP 3 193 991 B1**
(45) Date de publication et mention de la délivrance du brevet: **10.10.2018**
(21) Numéro de dépôt: 15794812.6
(22) Date de dépôt: 18.09.2015
(51) Int. Cl.: A61M 16/00, A61B 5/08, A61B 5/113, A61B 5/103

(54) **DISPOSITIF DE RÉGLAGE DE LA PRESSION D'UN GAZ RESPIRABLE**
VORRICHTUNG ZUR DRUCKREGELUNG EINES ATEMGASES
DEVICE FOR PRESSURE REGULATION OF A BREATHABLE GAS

(30) Priorité: 19.09.2014 BE 201400714
(43) Date de publication de la demande: 26.07.2017
(73) Titulaire: Nomics, 4031 Liège (Angleur) (BE)
(72) Inventeur: ANSAY, Pierre, 4031 Liège (BE); BECKERS, Bernard, 4031 Liège (BE); BOREL, Jean-Christian, 4031 Liège (BE); PEPIN, Jean-Louis, 4031 Liège (BE)
(74) Mandataire: Quintelier, Claude
(86) Numéro de dépôt international: PCT/BE2015/000045
(87) Numéro de publication internationale: WO 2016/041025

(56) Documents cités:
- EP-A1- 2 478 839
- EP-A2- 0 722 747
- EP-B1- 1 716 387
- WO-A1-2005/063323
- WO-A1-2010/088543
- WO-A2-2012/012835

## Description

La présente invention concerne un dispositif de réglage de l'apport d'un gaz respirable fourni en assistance respiratoire à un patient par un appareil d'assistance ventilatoire, lequel dispositif comprend une première entrée agencée pour recevoir un signal de fuite produit, en cas de détection d'une fuite, par un organe de détection de fuite de gaz fourni par l'appareil d'assistance ventilatoire, lequel dispositif est agencé pour régler l'apport du gaz respirable au patient en réponse au signal de fuite reçu.

Un tel dispositif de réglage est connu de la demande de brevet internationale WO 2010/088543. Le dispositif est généralement utilisé dans un appareil d'assistance ventilatoire, agencé pour fournir un gaz respirable en assistance respiratoire à un patient qui souffre d'insuffisances respiratoires ou de troubles respiratoires pendant le sommeil. L'appareil fait usage d'une interface, généralement constituée par un masque qui est placé sur le visage du patient. Le dispositif de réglage dont est équipé l'appareil connu, reçoit un signal de fuite indiquant qu'une fuite de gaz qui est fourni s'est produite, par exemple dû à un déplacement de l'interface. Le dispositif de réglage est agencé pour régler la fourniture du gaz respiratoire au patient en réponse au signal de fuite reçu. Ainsi le dispositif de réglage peut maintenir la pression du gaz fourni lorsqu'une fuite a été constatée.

Un désavantage du dispositif de réglage connu est que l'origine de la fuite est insuffisamment connue, en particulier lorsque la fuite est occasionnée par le patient même, par exemple lorsque la fuite est la cause d'un mouvement de la bouche du patient. Le dispositif connu n'est en effet pas en mesure de distinguer si la fuite est par exemple due à un déplacement de l'interface ou à un mouvement de la bouche du patient. Ceci peut alors mener à un réglage qui n'est pas toujours approprié.

L'invention a pour but de réaliser un dispositif de réglage de rapport du gaz respirable qui est en mesure de mieux reconnaître l'origine de la fuite et ainsi de mieux régler l'apport du gaz en cas de fuite.

A cette fin un dispositif de réglage suivant l'invention est caractérisé en ce que le dispositif comporte une deuxième entrée agencée pour recevoir un signal de mesure des mouvements de la bouche du patient produit par un dispositif de mesure des mouvements de la bouche, lequel dispositif de réglage comporte une unité de traitement agencée pour produire un signal de synchronisation en synchronisant dans le temps le signal de mesure des mouvements de la bouche du patient et le signal de fuite et pour constater, sous contrôle du signal de synchronisation, si une variation de la valeur du signal de mesure des mouvements de la bouche du patient s'est produite et produire un premier signal de contrôle lors d'une constatation qu'une variation de la valeur du signal de mesure des mouvements de la bouche du patient s'est produite, laquelle unité de traitement est agencée pour produire, sous contrôle du premier signal de contrôle, un taux de modification indiquant la modification à apporter à l'apport du gaz respirable fourni par l'appareil d'assistance ventilatoire. Le signal de mesure de variation d'ouverture de la bouche permet de déterminer des mouvements prédéfinis de la bouche indiquant par exemple la présence d'une apnée ou d'un autre trouble respiratoire du sommeil ou de l'éveil du patient En analysant si le signal de fuite coïncide dans le temps avec une variation de la distance d'ouverture de la bouche ou avec un mouvement prédéfini de la bouche, il est possible d'établir qu'il y a un lien entre l'occurrence de la fuite et le mouvement de la bouche. La production du premier signal de contrôle, qui a lieu lorsque l'unité de traitement a constaté ladite coïncidence dans le temps, permet d'également modifier l'apport du gaz respirable en fonction de la connaissance que la fuite a été occasionnée par exemple par un trouble respiratoire du sommeil ou une apnée du patient.

Une première forme de réalisation préférentielle du dispositif suivant l'invention est caractérisée en ce que l'unité de traitement est agencée pour vérifier si la variation de la valeur du signal de mesure des mouvements présente une oscillation dans le temps, et pour incorporer dans le taux de modification une augmentation d'apport du gaz lors de la constatation que la valeur du signal de mesure des mouvements oscille dans le temps. La présence d'une oscillation dans le temps dans le signal de mesure de variation de distance indique que le patient doit faire des efforts pour respirer et qu'une augmentation des résistances de voies respiratoires a eu lieu chez le patient. Si tel est le cas, une augmentation de l'apport du gaz s'impose.

Une deuxième forme de réalisation préférentielle du dispositif suivant l'invention est caractérisée en ce qu'elle comporte une mémoire agencée pour y stocker temporairement les signaux de synchronisation et les signaux de mesure de mouvement. Ceci permet de stocker les signaux de fuite et de mesure de distance pour ainsi permettre une analyse ultérieure.

De préférence l'unité de traitement est agencée pour vérifier si après avoir produit le signal de contrôle la valeur du signal de mesure de variation des mouvements reste sensiblement stable dans le temps et pour produire un deuxième signal de contrôle lorsqu'il a été constaté que la valeur du signal de variation de mesure des mouvements reste sensiblement stable dans le temps, laquelle unité de traitement est agencée pour incorporer dans le taux de modification une diminution de l'apport du gaz sous contrôle du deuxième signal de contrôle. Ceci permet de constater que la fuite est due à par exemple un changement de position du patient et qu'il est alors souhaitable de diminuer rapport du gaz.

L'invention concerne également un appareil d'assistance ventilatoire comprenant un dispositif de réglage suivant l'invention.

L'invention sera maintenant décrite plus en détails à l'aide des dessins qui illustrent une forme de réalisation d'un dispositif de réglage et d'un appareil d'assistance ventilatoire selon l'invention. Dans les dessins :
la figure 1 montre un patient accouplé à un appareil d'assistance ventilatoire;
la figure 2 illustre schématiquement le dispositif de réglage selon l'invention;
la figure 3 illustre à l'aide d'un organigramme une première forme de fonctionnement du dispositif de réglage suivant l'invention;
la figure 4 illustre à l'aide d'organigramme une deuxième forme de fonctionnement du dispositif de réglage suivant l'invention;
les figures 5 et 6 illustrent des exemples des signaux de fuite et de mesure de distance fournis au dispositif de réglage suivant l'invention.

Dans les dessins une même référence a été attribuée à un même élément ou à un élément analogue.

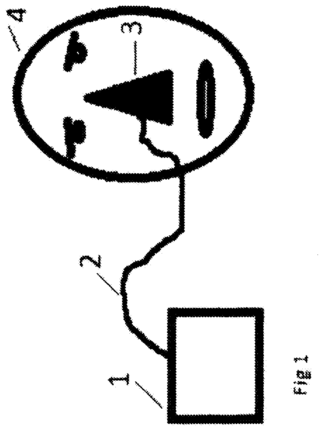

La figure 1 montre un patient 4 accouplé à un appareil 1 d'assistance ventilatoire, Le patient est accouplé à l'appareil à l'aide d'une interface 3 reliée à l'appareil à l'aide d'un tube d'alimentation 2. L'appareil d'assistance ventilatoire fournit un gaz respirable en assistance respiratoire au patient Ces appareils sont souvent dénommés par le terme CPAP (Continuous Positive Airway Pressure) ou Bilevel Positive Airway Pressure (B-PAP) et ils sont utilisés pour le traitement des syndromes d'apnées du sommeil ou de certains types d'insuffisances respiratoires. L'appareil est agencé pour insuffler un gaz respirable sous pression dans les voies aériennes supérieures du patient L'interface 3 entre le patient et l'appareil est formée en général par un masque placé sur le visage, en particulier sur le nez et/ou la bouche, du patient.

L'appareil d'assistance ventilatoire est muni de logiciels embarqués qui enregistrent, lors de l'utilisation, un nombre de signaux à chaque cycle respiratoire du patient, comme le débit respiratoire et la pression dans l'interface. En particulier, l'appareil d'assistance est équipé d'un organe de détection de fuite (nom repris dans le dessin) pour détecter une fuite du gaz respirable fourni au patient. Cet organe de détection de fuite va produire un signal de fuite lors de la détection d'une fuite au niveau de l'apport du gaz.

L'assistance ventilatoire par pression positive permet de garder la perméabilité des voies aériennes du patient au cours du sommeil ou/et de réduire le travail respiratoire et/ou de corriger une instabilité de la respiration. Si l'interface n'est pas correctement adaptée, une fuite peut survenir. Le type de fuite est «patient-indépendante», c'est-à-dire indépendante des mouvements de la bouche. Une seconde origine des fuites est dite d'origine «patient-dépendante», car associée au patient et non à son interface. Le positionnement du patient ou de sa tête peut engendrer une fuite, une ouverture de bouche permettant au gaz de s'échapper en partie ou un mouvement spécifique de la bouche peut également engendrer une fuite. Connaître l'origine des fuites c'est permettre une meilleure adaptation de la thérapie et d'améliorer l'observance.

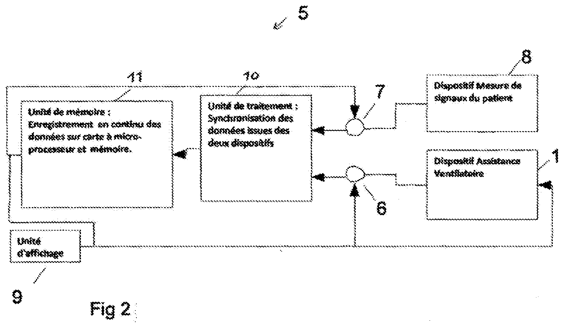

La figure 2 illustre schématiquement le dispositif de réglage selon l'invention. Ce dispositif de réglage est soit intégré dans l'appareil d'assistance, soit est formé par une unité séparée, qui peut être reliée à l'appareil d'assistance ventilatoire. Le dispositif 5 comporte une première entrée 6, agencée pour recevoir le signal de fuite produit par l'organe de détection de fuite de l'appareil 1 d'assistance respiratoire. Le dispositif comporte également une deuxième entrée 7 agencée pour recevoir un signal de mesure des mouvements de la bouche du patient. Ce signal de mesure des mouvements est produit par un dispositif 8 de mesure de mouvements de la bouche du patient. Un tel dispositif de mesure est par exemple décrit dans le brevet EP 1716387.

La première et la deuxième entrée sont reliées à une unité de traitement 10, par exemple formée par un microprocesseur. L'unité de traitement est reliée à la première et la deuxième entrée pour recevoir le signal de fuite et le signal de mesure des mouvements de la bouche. L'unité de traitement est agencée pour produire un signal de synchronisation en synchronisant dans le temps le signal de mesure de mouvements et le signal de fuite.

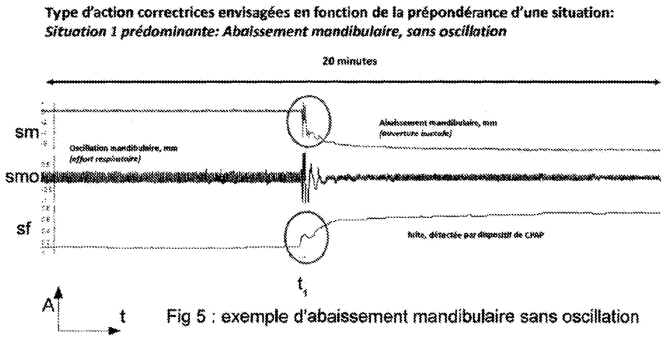

La figure 5 illustre un graphique avec un exemple d'un signal sm de mesure des mouvements de la bouche et d'un signal de fuite sf. L'axe horizontal du graphique indique le temps et l'axe vertical indique l'amplitude du signal. Le signal sm est un signal qui indique un abaissement de la bouche du patient sans pourtant qu'une oscillation se produit. On voit en effet qu'au temps t1 une chute dans le signal, qui indique qu'un mouvement de la bouche s'est produit, en particulier que la bouche du patient s'est ouverte. Pratiquement au même moment t1, on constate que l'amplitude du signal sf a changé, ce qui indique qu'une fuite du gaz fourni au patient a été constatée. On constate donc qu'il existe une corrélation dans le temps entre le mouvement de la bouche et l'occurrence d'une fuite dans la fourniture du gaz respirable au patient.

La figure 5 montre également un signal smo qui représente l'oscillation mandibulaire du patient, qui est une autre façon d'exprimer les mouvements de la bouche du patient. Dans ce signal de mesure des mouvements de la bouche on constatera également qu'aux alentours du moment t1 l'amplitude de signal change.

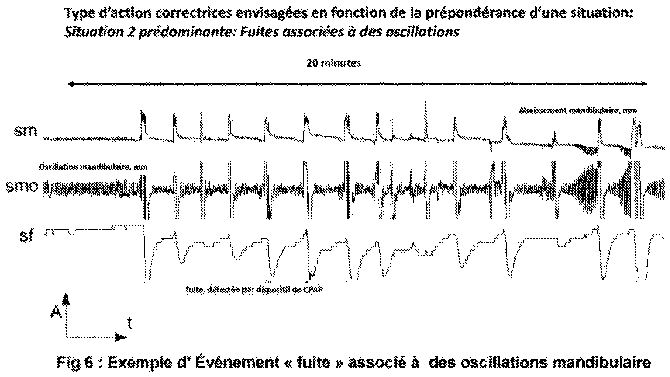

La figure 6 montre également des signaux sm, smo et sf. Les signaux illustrés à la figure 6 se distinguent de ceux de la figure 5 par la présence de plusieurs oscillations dans le signal. Les oscillations dans les signaux sm et smo indiquent que la bouche du patient est particulièrement instable et qu'elle oscille entre un état ouvert et un état fermé. Les signaux sm et smo indiquent un état apnée chez le patient qui fait de considérables efforts pour respirer. En faisant une corrélation temporelle entre les signaux sm ou smo et sf on constatera que le signal sf est également très instable, ce qui indique qu'il y a une oscillation dans la fuite détectée par l'organe de détection de fuite. La figure 6 montre également qu'il y a une coïncidence temporelle entre la détection d'une fuite et le mouvement de la bouche et que les fuites varient suivant une fréquence comparable à celle à laquelle la bouche varie.

Lorsque la fuite n'est pas liée à un mouvement de la bouche mais à un mauvais positionnement de l'interface sur le patient, les signaux sm et smo restent sensiblement stable et lorsque l'interface est repositionnée correctement, le signal de fuite sf chute en amplitude.

La présente invention est basée sur cette constatation qu'il existe une corrélation dans le temps entre un mouvement de la bouche et l'occurrence d'une fuite dans le gaz fourni au patient. C'est la raison pour laquelle l'unité de traitement 10 est agencée pour synchroniser dans le temps le signal de mouvements de la bouche et le signal de fuite. C'est cette corrélation qui va permettre d'établir que l'origine de la fuite est due à un mouvement de la bouche.

Comme mentionné ci-dessus, l'unité de traitement est agencée pour produire le signal de synchronisation. Etant entendu que le signal de fuite n'est produit que lors de l'apparition d'une fuite, le fait de synchroniser dans le temps le signal de fuite avec le signal de mesure de mouvement de la bouche va permettre d'établir si un lien temporel existe entre une fuite du gaz et un mouvement de la bouche.

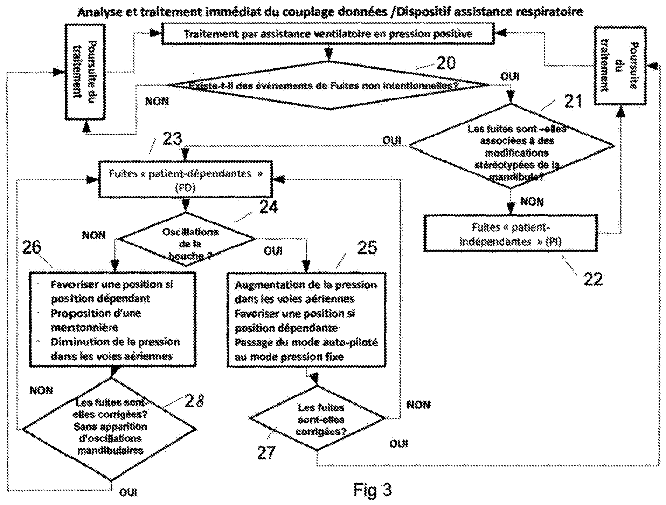

L'unité de traitement va produire (20) le signal de synchronisation chaque fois qu'elle reçoit le signal de fuite. Ceci est illustré par l'étape 20 repris dans l'organigramme illustré à la figure 3 et qu'illustre une première forme de fonctionnement du dispositif suivant l'invention. Le signal de synchronisation est par exemple produit en couplant le signal de fuite au signal de mesure des mouvements de la bouche. L'unité de traitement de données est également agencée pour constater (21), sous contrôle du signal de synchronisation, si une variation de la valeur du signal de mesure des mouvements de la bouche (sm, smo) s'est produite. Lorsque l'unité de traitement constate que le signal de mesure de mouvement ne comporte pas une variation (21, N) cela signifie que la fuite n'est pas occasionnée par le mouvement de la bouche. Dans ce dernier cas l'unité de traitement annule (22) le signal de synchronisation.

Par contre, si sous contrôle du signal de synchronisation il est constaté (21, O) que le signal de mesure de mouvement de la bouche a varié, parce que la bouche du patient s'est ouverte (figure 5 ou 6), l'unité de traitement va produire (23) un premier signal de contrôle. Ce dernier va à son tour déclencher la production d'un taux de modification indiquant la modification à apporter à l'apport du gaz respirable qui doit être modifié. Cette modification est par exemple réalisée en modifiant la pression sous laquelle le gaz est fourni ou en modifiant le réglage de la fréquence du gaz fourni. Par fréquence du gaz fourni on entend la fréquence à laquelle une quantité prédéterminée de gaz est fourni au patient.

De préférence le taux avec lequel l'apport de gaz est modifié est déterminé en fonction de la valeur du signal de mesure des mouvements de la bouche. Ainsi lors de l'étape 24 l'unité de traitement va vérifier si la variation de la valeur du signal de mesure des mouvements dépasse un seuil prédéterminé ou représente un mouvement prédéterminé de la bouche. Par mouvement prédéterminé de la bouche on entend par exemple un mouvement qui caractérise la présence d'une apnée ou d'un autre trouble respiratoire du sommeil ou un mouvement qui caractérise l'éveil du patient. Si tel n'est pas le cas le premier signal de contrôle n'est pas produit (26) et aucune modification est apportée à rapport de gaz. En effet, il est possible que le dispositif de mesure du mouvement de la bouche constate qu'un faible mouvement de la bouche qui n'a occasionné qu'une faible fuite, mais que la fuite a déjà disparu. Dans ce cas il n'est pas nécessaire de modifier l'apport de gaz.

La valeur du signal de mesure des mouvements est de préférence prise en compte lors de la détermination de la modification de l'apport de gaz. En effet, comme illustré aux figures 6 et 5 le mouvement de la bouche peut osciller ou pas. A cette fin l'unité de traitement va vérifier (24) si la variation de la valeur du signal de mesure des mouvements présente une oscillation dans le temps. Ceci est par exemple réalisé en surveillant si, après la production d'un signal de synchronisation, d'autres signaux de synchronisation sont produits. En effet, comme le montre la figure 6, si la bouche oscille, le signal de fuite va aussi osciller. Par contre si la bouche n'oscille pas, comme illustré à la figure 5, le signal de fuite reste stable. Cette analyse permet donc de constater si la fuite est causée par une oscillation de la bouche du patient.

Si le signal de mesure du mouvement oscille (24,O), le taux de modification va incorporer une augmentation (25) de l'apport de gaz. Si par contre le signal de mesure reste sensiblement stable (28) dans le temps, l'unité de traitement va produire un deuxième signal de contrôle. Sous contrôle de ce deuxième signal de contrôle l'unité de traitement va adapter le taux de modification de l'apport de gaz, par exemple en y incorporant une baisse de la pression de gaz afin de réduire la fuite.

Après avoir modifié l'apport du gaz au patient l'unité de traitement va vérifier (27) si la fuite est compensée. Si tel n'est pas le cas le procédé est repris à l'étape (24).

Le dispositif suivant l'invention est de préférence équipé d'une mémoire (11) reliée à l'unité de traitement (10) et agencée pour y stocker temporairement les signaux de synchronisation et les signaux de mesure du mouvement. Le cas échéant le dispositif est relié à une unité d'affichage (9) qui permet d'afficher les signaux stockés dans la mémoire.

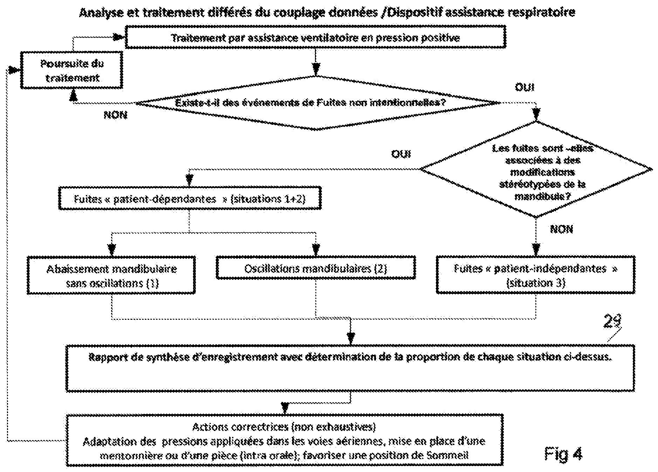

Suivant une autre forme de réalisation illustrée à la figure 4, l'unité de traitement est agencée pour fournir (29) un rapport de synthèse d'enregistrement avec détermination de la proportion de chaque situation. Ce rapport reprend ainsi les signaux de fuite et de mesure de mouvement de la bouche et suggère en fonction de ces signaux des actions à prendre en considération par un clinicien. Ce rapport de synthèse est, le cas échéant, disponible sur un écran distant ou peut être envoyé par un réseau de communication vers un clinicien.

## Revendications

1. Dispositif (5) de réglage de l'apport d'un gaz respirable fourni en assistance respiratoire à un patient (4) par un appareil (1) d'assistance ventilatoire, lequel dispositif (5) comprend une première entrée (6) agencée pour recevoir un signal de fuite produit, en cas de détection d'une fuite, par un organe de détection de fuite du gaz fourni par l'appareil (1) d'assistance ventilatoire, lequel dispositif (5) est agencé pour régler l'apport du gaz respirable au patient (4) en réponse au signal de fuite reçu, **caractérisé en ce que** le dispositif (5) comporte une deuxième entrée (7) agencée pour recevoir un signal de mesure des mouvements de la bouche du patient (4) produit par un dispositif (8) de mesure des mouvements de la bouche, lequel dispositif (5) de réglage comporte une unité de traitement (10) agencée pour produire un signal de synchronisation en synchronisant dans le temps le signal de mesure des mouvements de la bouche et le signal de fuite et pour constater, sous contrôle du signal de synchronisation, si une variation de la valeur du signal de mesure des mouvements de la bouche s'est produite et produire un premier signal de contrôle lors d'une constatation qu'une variation de la valeur du signal de mesure des mouvements de la bouche s'est produite, laquelle unité de traitement (10) est agencée pour produire, sous contrôle du premier signal de contrôle, un taux de modification indiquant la modification à apporter à l'apport du gaz respirable fourni par l'appareil (1) d'assistance ventilatoire.

2. Dispositif (5) de réglage selon la revendication 1, **caractérisé en ce que** l'unité de traitement (10) est agencée pour vérifier si la variation de la valeur du signal de mesure des mouvements de la bouche dépasse un seuil prédéterminé ou représente un mouvement prédéfini de la bouche et pour produire le premier signal de contrôle si la valeur du signal de mesure dépasse le seuil prédéterminé ou représente un mouvement prédéfini de la bouche.

3. Dispositif (5) de réglage selon les revendications 1 ou 2, **caractérisé en ce que** l'unité de traitement (10) est agencée pour déterminer le taux de modification également en fonction de la valeur du signal de mesure des mouvements de la bouche.

4. Dispositif (5) de réglage selon l'une des revendications 1 à 3, **caractérisé en ce que** l'unité de traitement (10) est agencée pour vérifier si la variation de la valeur du signal de mesure des mouvements de la bouche présente une oscillation dans le temps, et pour incorporer une augmentation de l'apport de gaz dans le taux de modification lors de la constatation que la valeur du signal de mesure des mouvements de la bouche oscille dans le temps.

5. Dispositif (5) de réglage selon l'une des revendications 1 à 4, **caractérisé en ce qu'**il comporte une mémoire (11) agencée pour y stocker temporairement les signaux de synchronisation et les signaux de mesure de mouvement de la bouche.

6. Dispositif (5) de réglage selon l'une des revendications 1 à 5, **caractérisé en ce que** l'unité de traitement (10) est agencée pour vérifier si après avoir produit le signal de contrôle la valeur du signal de mesure de variation des mouvements de la bouche reste sensiblement stable dans le temps et pour produire un deuxième signal de contrôle lorsqu'il a été constaté que la valeur du signal de variation de mesure des mouvements de la bouche reste sensiblement stable dans le temps, laquelle unité de traitement (10) est agencée pour adapter le taux de modification de l'apport du gaz sous contrôle du deuxième signal de contrôle.

7. Dispositif (5) de réglage selon l'une des revendications 1 à 6, **caractérisé en ce qu'**il est agencé pour régler l'apport du gaz par un réglage de la pression du gaz fourni.

8. Dispositif (5) de réglage selon l'une des revendications 1 à 7, **caractérisé en ce qu'**il est agencé pour régler l'apport du gaz par un réglage de la pression à l'inspiration et à l'expiration du gaz fourni.

9. Dispositif (5) de réglage selon l'une des revendications 1 à 8, **caractérisé en ce qu'**il est agencé pour régler l'apport du gaz par un réglage de la fréquence à laquelle le gaz est fourni.

10. Appareil (1) d'assistance ventilatoire comprenant un dispositif (5) de réglage selon l'une des revendications 1 à 9.

## Patentansprüche

1. Vorrichtung (5) zum Einstellen der Zufuhr eines Atemgases, das zur Beatmungsunterstützung an einen Patienten (4) von einem Beatmungsunterstützungsgerät (1) geliefert wird, wobei die Vorrichtung (5) einen ersten Eingang (6) umfasst, der eingerichtet ist, ein Lecksignal zu empfangen, das im Falle der Erfassung eines Lecks des Gases erzeugt wird, das von der Beatmungsunterstützungsvorrichtung (1) geliefert wird, wobei die Vorrichtung (5) eingerichtet ist, um die Zufuhr von Atemgas zu dem Patienten (4) als Antwort auf das empfangene Lecksignal einzustellen, **dadurch gekennzeichnet, dass** die Vorrichtung (5) einen zweiten Eingang (7) aufweist, der eingerichtet ist, um ein Bewegungsmesssignal des Mundes des Patienten (4) zu empfangen, das von einer Vorrichtung (8) zum Messen von Bewegungen des Mundes erzeugt wird, wobei die Vorrichtung (5) zum Einstellen eine Verarbeitungseinheit (10) aufweist, die eingerichtet ist, um ein Synchronisationssignal zu erzeugen durch Synchronisierung mit der Zeit des Bewegungsmesssignals des Mundes und des Leck Signals, und um unter Steuerung des Synchronisationssignals festzustellen, ob eine Änderung des Werts des Bewegungsmesssignals des Mundes aufgetreten ist, und ein erstes Steuersignal zu erzeugen bei die Feststellung dass eine Änderung des Werts des Bewegungsmesssignals des Mundes aufgetreten ist, wobei die Verarbeitungseinheit (10) eingerichtet ist, um unter der Steuerung des ersten Steuersignals eine Modifizierungsrate zu erzeugen, die die Modifizierung angibt, die an der Zufuhr von Atemgas, das von dem Beatmungsunterstützungsgerät (1) geliefert wird, vorgenommen werden soll.

2. Vorrichtung (5) zum Einstellen nach Anspruch 1, **dadurch gekennzeichnet, dass** die Verarbeitungseinheit (10) eingerichtet ist, um zu überprüfen, ob die Änderung des Wertes des Bewegungsmesssignals des Mundes einen vorgegebenen Schwellenwert überschreitet oder eine vordefinierte Bewegung des Mundes darstellt, und um das erste Steuersignal zu erzeugen, wenn der Wert des Messsignals den vorgegebenen Schwellenwert überschreitet oder eine vordefinierte Bewegung des Mundes darstellt.

3. Vorrichtung (5) zum Einstellen nach Anspruch 1 oder 2, **dadurch gekennzeichnet, dass** die Verarbeitungseinheit (10) eingerichtet ist, die Modifizierungsrate auch in Abhängigkeit von dem Wert des Bewegungsmesssignals des Mundes zu bestimmen.

4. Vorrichtung (5) zum Einstellen nach einem der Ansprüche 1 bis 3, **dadurch gekennzeichnet, dass** die Verarbeitungseinheit (10) eingerichtet ist, zu überprüfen, ob die Änderung des Wertes des Bewegungsmesssignals des Mundes eine Oszillation mit der Zeit aufweist, und um eine Erhöhung der Zufuhr des Gases in die Modifizierungsrate zu inkorporieren bei die Feststellung dass der Wert des Bewegungsmesssignals des Mundes mit der Zeit oszilliert.

5. Vorrichtung (5) zum Einstellen nach einem der Ansprüche 1 bis 4, **dadurch gekennzeichnet, dass** sie einen Speicher (11) aufweist, der eingerichtet ist, um darin die Synchronisationssignale und die Bewegungsmesssignale des Mundes zeitweilig zu speichern.

6. Vorrichtung (5) zum Einstellen nach einem der Ansprüche 1 bis 5, **dadurch gekennzeichnet, dass** die Verarbeitungseinheit (10) eingerichtet ist, um zu überprüfen, ob, nachdem das Steuersignal erzeugt wurde, der Wert des Messsignals der Änderung der Bewegung des Mundes mit der Zeit im Wesentlichen stabil bleibt, und um ein zweites Steuersignal zu erzeugen, wenn festgestellt wurde, dass der Wert des Messsignals der Änderung der Bewegungen des Mundes mit der Zeit im Wesentlichen stabil bleibt, wobei die Verarbeitungseinheit (10) eingerichtet ist, die Modifizierungsrate der Zufuhr des Gases unter Steuerung des zweiten Steuersignals anzupassen.

7. Vorrichtung (5) zum Einstellen nach einem der Ansprüche 1 bis 6, **dadurch gekennzeichnet, dass** sie eingerichtet ist, die Zufuhr des Gases durch ein Einstellen des Drucks des gelieferten Gases einzustellen.

8. Vorrichtung (5) zum Einstellen nach einem der Ansprüche 1 bis 7, **dadurch gekennzeichnet, dass** sie eingerichtet ist, die Zufuhr des Gases durch ein Einstellen des Druckes bei der Einatmung und der Ausatmung des gelieferten Gases einzustellen.

9. Vorrichtung (5) zum Einstellen nach einem der Ansprüche 1 bis 8, **dadurch gekennzeichnet, dass** sie eingerichtet ist, die Zufuhr des Gases durch ein Einstellen der Frequenz einzustellen, mit der das Gas geliefert wird.

10. Beatmungsunterstützungsgerät (1), umfassend eine Vorrichtung (5) zum Einstellen nach einem der Ansprüche 1 bis 9.

## Claims

1. A breathable gas supply regulation device (5), said gas being supplied by a respiratory assistance apparatus (1) in respiratory assistance to a patient (4), which device (5) comprises a first input (6) provided for receiving a leakage signal produced, in case of a gas leakage detection, by a detection member provided to detect a leakage in the gas supplied by the respiratory assistance apparatus (1), which device (5) is provided for regulating the supply of breathable gas to the patient (4) in response to a received leakage signal, **characterised in that** the device (5) comprises a second input (7) provided for receiving a patient (4) mouth movement measurement signal produced by a mouth movement measurement device (8), which regulation device (5) comprises a processing unit (10) provided for generating a synchronisation signal by synchronising in time the mouth movement measurement signal and the leakage signal and for establishing, under control of the synchronisation signal, if a variation of the value of the mouth movement measurement signal occurred and for generating a first control signal when establishing that a variation of value of the mouth movement measurement signal occurred, which processing unit (10) is provided for generating under control of the first control signal a modification amount indicating the modification to be applied to the supply of breathable gas supplied by the respiratory assistance apparatus (1).

2. The regulation device (5) as claimed in claim1, **characterised in that** the processing unit (10) is provided for verifying if the variation of the value of the mouth movement measurement signal exceeds a predetermined threshold or represents a predefined mouth movement, and for generating the first control signal if the measurement signal exceeds the predetermined threshold or represents a predefined mouth movement.

3. The regulation device (5) as claimed in claim 1 or 2, **characterised in that** the processing unit (10) is provided for determining the modification amount also in function of the value of the mouth movement measurement signal.

4. The regulation device (5) as claimed in anyone of the claims 1 to 3, **characterised in that** the processing unit (10) is provided for verifying if the variation of the value of the mouth movement measurement signal presents an oscillation in time, and for incorporating an increase of the gas supply in the modification amount when establishing that the value of the mouth movement measurement signal oscillates in time.

5. The regulation device (5) as claimed in anyone of the claims 1 to 4, **characterised in that** it comprises a memory (11) provided for temporarily storing therein the synchronisation signals and the mouth movement measurement signals.

6. The regulation device (5) as claimed in anyone of the claims 1 to 5, **characterised in that** the processing unit (10) is provided for verifying if, after having generated the control signal, the value of the measurement signal of the mouth movement variations remains essentially stable in time and for generating a second control signal when it has been established that the value of the measurement signal of the mouth movement variations remains essentially stable in time, which processing unit (10) is provided for adapting the modification amount of the gas supply under control of the second control signal.

7. The regulation device (5) as claimed in anyone of the claims 1 to 6, **characterised in that** it is provided for regulating the gas supply by a pressure control of the supplied gas.

8. The regulation device (5) as claimed in anyone of the claims 1 to 7, **characterised in that** it is provided for regulating the gas supply by a pressure control at inhalation and at exhalation of the supplied gas.

9. The regulation device (5) as claimed in anyone of the claims 1 to 8, **characterised in that** is provided for regulating the gas supply by a control of the frequency at which the gas is supplied.

10. Respiratory assistance apparatus (1) comprising a regulation device (5) as claimed in anyone of the claims 1 to 9.
